# EUROPÄISCHE PATENTSCHRIFT

(11) **EP 2 619 320 B1**
(45) Veröffentlichungstag und Bekanntmachung des Hinweises auf die Patenterteilung: **18.11.2015**
(21) Anmeldenummer: 11760486.8
(22) Anmeldetag: 23.09.2011
(51) Int. Cl.: C12Q 1/68, C12N 1/06, C12M 1/33

(54) **REAGENZ ZUM AUFSCHLUSS VON ZELLMATERIAL MIT VOLLSTÄNDIG INTEGRIERTEM INTERNEM STANDARD**
REAGENT FOR THE DISRUPTION OF CELL MATERIAL HAVING A COMPLETELY INTEGRATED INTERNAL STANDARD
RÉACTIF DE DISSOCIATION DE MATÉRIEL CELLULAIRE COMPRENANT UN ÉTALON INTERNE ENTIÈREMENT INTÉGRÉ

(30) Priorität: 24.09.2010 EP 10010447
(43) Veröffentlichungstag der Anmeldung: 31.07.2013
(73) Patentinhaber: Qiagen GmbH, 40724 Hilden (DE)
(72) Erfinder: HECKEL, Dirk, F-38410 St. Martin d' Uriage (FR); BELLER, Katharina, 40724 Hilden (DE)
(74) Vertreter: f & e patent
(86) Internationale Anmeldenummer: PCT/EP2011/066562
(87) Internationale Veröffentlichungsnummer: WO 2012/038523

(56) Entgegenhaltungen:
- FRANZISKA DIETRICH ET AL: "Fine-tuning of a three-dimensional microcarrier-based angiogenesis assay for the analysis of endothelial-mesenchymal cell co-cultures in fibrin and collagen gels", ANGIOGENESIS, KLUWER ACADEMIC PUBLISHERS, DO, Bd. 9, Nr. 3, 19. Oktober 2006 (2006-10-19), Seiten 111-125, XP019432800, ISSN: 1573-7209, DOI: DOI:10.1007/S10456-006-9037-X
- WOHLER W ET AL: "Large scale culturing of normal diploid cells on glass beads using a novel type of culture vessel", EXPERIMENTAL CELL RESEARCH, ACADEMIC PRESS, US, Bd. 74, Nr. 2, 1. Oktober 1972 (1972-10-01), Seiten 571-573, XP024792116, ISSN: 0014-4827, DOI: DOI:10.1016/0014-4827(72)90419-3 [gefunden am 1972-10-01]
- KALISH D I ET AL: "MEMBRANE ISOLATION OF POLYLYSINE-COATED GLASS BEADS ASYMMETRY OF BOUND MEMBRANE", BIOCHIMICA ET BIOPHYSICA ACTA, ELSEVIER, NL, Bd. 506, Nr. 1, 1. Januar 1978 (1978-01-01), Seiten 97-110, XP009016725, ISSN: 0006-3002, DOI: DOI:10.1016/0005-2736(78)90437-6
- SANFORD GARY L ET AL: "Three-dimensional growth of endothelial cells in the microgravity-based rotating wall vessel bioreactor", IN VITRO CELLULAR & DEVELOPMENTAL BIOLOGY - ANIMAL, SPRINGER, GERMANY, UNITED STATES, Bd. 38, Nr. 9, 1. Oktober 2001 (2001-10-01), Seiten 493-504, XP009144922, ISSN: 1071-2690
- GARY R KLINEFELTER AND LARRY L EWING: "Optimizing testosterone production by purified adult rat Leydig cells in vitro", IN VITRO CELLULAR AND DEVELOPMENTAL BIOLOGY, THE ASSOCIATION, GAITHERSBURG, MD, US, Bd. 24, Nr. 6, 1. Januar 1988 (1988-01-01) , Seiten 545-549, XP009144788, ISSN: 0883-8364
- DOTY A ET AL: "Development and validation of an independent positive control for nucleic acid tests for microorganisms that cause septicemia", CLINICAL CHEMISTRY AND LABORATORY MEDICINE, WALTER DE GRUYTER & CO, BERLIN, NEW YORK, Bd. 44, Nr. 4, 1. April 2006 (2006-04-01), Seite A5, XP009144950, ISSN: 1434-6621, DOI: DOI:1515/CCLM.2006.169 [gefunden am 2006-04-07]

### SEQUENCE LISTING

<110> QIAGEN GmbH
<120> Reagenz zum Aufschluss von Zellmaterial mit vollständig integriertem internem Standard
<130> PA346-PCT
<160> 3
<170> PatentIn version 3.3
<210> 1
   <211> 23
   <212> DNA
   <213> Artificial Sequence
<220>
   <223> Synthetic DNA
<400> 1
   cactacggtg ctgaagcgac aaa 23
<210> 2
   <211> 19
   <212> DNA
   <213> Artificial Sequence
<220>
   <223> Synthetic DNA
<400> 2
   ccaggcaaat ccggaaaac 19
<210> 3
   <211> 22
   <212> DNA
   <213> Artificial Sequence
<220>
   <223> Synthetic DNA
<400> 3
   gtacgatttg atgttacctg at 22

## Beschreibung

Die vorliegende Erfindung betrifft ein Reagenz zum Aufschluss von Zellmaterial, enthaltend einen vollständig in das Reagenz integrierten internen Standard zur Kontrolle und Beurteilung der Vollständigkeit des Aufschlusses des Zellmaterials und zur Kontrolle und Beurteilung der Effizienz nachfolgender Schritte wie Probenaufbereitung, Extraktion, Konzentration, Isolierung, Reinigung, reverse Transkription, Amplifikation und Detektion der aus den aufgeschlossenen Zellen erhaltenen Zellbestandteile oder einer Kombination mehrerer oder aller dieser Schritte, umfassend Beads, deren Oberfläche mit Referenzzellen als Standard beschichtet ist.

Die Isolierung, Charakterisierung und Analyse intrazellulärer Bestandteile, vor allem von Proteinen und Nukleinsäuren (Ribonukleinsäuren, RNA und Desoxyribonukleinsäuren, DNA), ist von großer Bedeutung für die moderne Molekularbiologie. Spätestens seit der Entdeckung der Polymerasekettenreaktion (PCR) im Jahre 1983 ist eine Vielzahl von Nukleinsäurediagnostikverfahren entwickelt worden, die beispielsweise zum Nachweis von Krankheiten und Krankheitserregern genutzt werden.

Der erste Schritt zur Isolierung und Untersuchung intrazellulärer Bestandteile ist der Zellaufschluss (die Lyse der Zellen), um die intrazellulären Bestandteile aus der Zelle freizusetzen. Die Vorgehensweise und die Effizienz des Aufschlusses haben einen entscheidenden Einfluss auf die Gesamtmenge der erhaltenen Bestandteile, ihre biologische Aktivität, Integrität und die Kontamination des erhaltenen Materials. Im Laufe der Zeit ist eine Vielzahl von Verfahren zur Zelllyse entwickelt worden, von denen vor allem die enzymatische Zelllyse unter Abbau von Zellwandkomponenten durch Enzyme, die chemische Zelllyse unter Verwendung von Detergenzien, Basen und chaotropen Agenzien sowie die mechanische Zelllyse, bei der die Zellwand durch mechanische Belastung aufgebrochen wird, von Bedeutung sind. Beispielsweise für Pilze-, Hefe-, Bakterien-, Sporen- und Pflanzenzelle, die stabile Zellwände besitzen, eignen sich vor allem mechanische Methoden. Rein mechanische Aufschlussmethoden wie z. B. die Verwendung von Beads (Kügelchen aus festem Material wie Glas, Stahl etc.) zeichnen sich gegenüber anderen bekannten Methoden dadurch aus, dass auch Zellen mit sehr harter Zellwand in kurzer Zeit aufgeschlossen werden können, des Weiteren große Zellmengen bearbeitet werden können und dass durch den Aufschluss keine Additive In die Probe eingebracht werden, die bei der anschließenden Isolierung, Analyse und Charakterisierung der Zell bestandteile möglicherweise störend wirken könnten.

Da der Prozess des Aufschlusses von entscheidender Bedeutung für sämtliche nachfolgenden Schritte wie beispielsweise die Isolierung, Reinigung, Amplifikation, Analytik und Detektion von Zellbestandteilen ist, ist es zweckmäßig, einen Standard zu verwenden, der die Kontrolle und Beurteilung der Vollständigkeit des Aufschlusses des Zellmaterials erlaubt. Vorteilhafterweise sollte ein solcher Standard nicht nur die Beurteilung der Effizienz des Aufschlusses selbst ermöglichen, sondern auch eine Kontrolle nachfolgender Schritte wie Probenaufbereitung, Extraktion, Konzentration, Isolierung, Reinigung, reverse Transkription, Amplifikation und/oder Detektion der aus den aufgeschlossenen Zellen erhaltenen Zellbestandteilen erlauben.

Ein derartiges Kontrollkonzept ist beispielweise die kommerziell erhältliche ACCURUN® 500 bacterial/fungal Sepsis Kontrolle (BBI-Diagnostics, West Bridgewater, Plymouth, USA), welches auch in Clin Chem, Med 2006; 44(4): A8 von Kessler, H.H. et al. beschrieben wird.. Hierbei handelt es sich um ein externes Kontrollkonzept unter Verwendung einer Referenzprobe, bei dem drei Kontrollorganismen, nämlich ein grampositives Bakterium (*S*. *aureus*), ein gramnegatives (*P. aeruginosa*) und ein Hefepilz (*C*. *albicans*), welche zuvor durch Hitzebehandlung inaktiviert wurden, in negatives Humanplasma eingebracht sind. Diese Referenzprobe wird parallel zu dem zu untersuchenden Zellmaterial (Probenzellmaterial) unter Anwendung des Verfahrens, welches auch für das Probenzellmaterial verwendet wird, analysiert. Ein genereller Nachteil externer Kontrollen ist allerdings, dass hierbei singulär auftretende Fehler, wie beispielsweise Pipettierfehler, die lediglich das Probenzellmaterial betreffen, durch das Kontrollsystem nicht erfasst werden können.

US 2007/0015139 A1 beschreibt ein Kontrollkonzept zur Validierung der Ergebnisse in Nukleinsäuretestverfahren, bei dem der zu untersuchenden Probe vor der Lyse des Zellmaterials Mikroorganismen hinzugesetzt werden. Hierbei handelt es sich insofern um einen internen Standard, als dass das Probenzellmaterial und das Referenzzellmaterial gemeinsam in einem Reaktionsgefäß prozessiert werden. Allerdings muss auch für dieses Kontrollkonzept der Standard in einem separaten Verfahrensschritt zusätzlich zu den Reagenzien zur Probe hinzugegeben werden, was ebenfalls eine potentielle Fehlerquelle darstellt.

Die Aufgabe der vorliegenden Erfindung bestand darin, ein Reagenz zum Aufschluss von Zellmaterial bereitzustellen, das einen vollständig in das Reagenz integrierten internen Standard zur Kontrolle und Beurteilung der Vollständigkeit des Aufschlusses des Zellmaterials und nachfolgender Schritte enthält.

Überraschenderweise ist nun gefunden worden, dass die zum Aufschluss des Zellmaterials verwendeten Beads mit eingetrockneten Referenzzellen beschichtet werden können, welche bei einem Aufschluss des Probenzellmaterials unter Verwendung der Beads gemeinsam mit dem Probenzellmaterial aufgeschlossen werden. Auf diese Weise wird ein vollständig in das Reagenz integrierter interner Standard zur Kontrolle und Beurteilung der Vollständigkeit des Aufschlusses des Zellmaterials und nachfolgender Schritte erhalten.

Die Beschichtung verschiedener Typen von Beads, insbesondere Dextranbeads und Glasbeads, mit Zellen ist grundsätzlich bekannt und beispielsweise von Dietrich, F. und Lelkes, P. I. in Angiogenesis (2006) 9:111-125; von Wöhler, W. et al. in Exptl Cell Res 74 (1972); von Kalish, D. I. in Biochimica et Biophysica Acta, 506 (1978) 97-119; von Sanford, G. L. et al. in In Vitro Cell Dev. Biol.-Animal 38:493-504, October 2002 und von Klinefelter, G. Y. und Ewing, L. L. in In Vitro Celll Dev. Biology Vol 24, 6 (6.6.1988) beschrieben.

Ein Gegenstand der vorliegenden Erfindung ist daher ein Reagenz zum Aufschluss von Zellmaterial (Probenzellmaterial), enthaltend einen vollständig in das Reagenz integrierten Standard zur Kontrolle und Beurteilung der Vollständigkeit des Aufschlusses des Zellmaterials und zur Kontrolle und Bewertung nachfolgender Schritte, umfassend mindestens einen Schritt ausgewählt aus Probenaufbereitung, Extraktion, Anreicherung, Isolierung, Reinigung, reverser Transkription, Amplifikation und Detektion der aus den aufgeschlossenen Zellen erhaltenen Zellbestandteile oder einer Kombination mehrerer oder aller dieser Schritte, umfassend Beads, an deren Oberfläche eingetrocknete Referenzzellen angelagert sind oder deren Oberfläche mit eingetrockneten Referenzzellen (Referenzzellmaterial) als Standard wenigstens teilweise beschichtet ist, wobei die Beads aus Glas, Metall, Keramik oder anderen porösen Materialien oder Wolframcarbid bestehen und die Größe der Beads 0.01 mm bis 2 mm beträgt. Eine zusätzliche Beschichtung mit einer Schutzhülle zur zusätzlichen Stabilisierung der auf den Beads aufgebrachten Referenzzellen, z. B. mit selbst assemblierenden Polymeren, ist ebenfalls denkbar.

Das Reagenz kann aus den Beads und den daran angelagerten Referenzzellen bestehen, oder es kann weitere Bestandteile enthalten, wie beispielsweise eine wässrige oder organische Flüssigkeit, bevorzugt Wasser, eine Pufferlösung, eine Nährlösung für die Zellen oder eine isotonische wässrige Lösung, bevorzugt Kochsalzlösung, oder - auch in getrocknetem Zustand - Pufferkomponenten, Salze, Nährstoffe für die Zellen oder ähnliches. Eine bevorzugte Ausführungsform ist, dass das Reagenz eine Suspension von an Beads angelagerten Zellen in bekannter Menge in einer wässrigen Lösung oder Wasser enthält.

Als Probenzellmaterial wird im Sinne der Erfindung das zu untersuchende Zellmaterial verstanden. Dieses Zellmaterial kann pflanzlichen, tierischen und/oder humanen Ursprungs sein, aus Gewebe- oder Zellkulturen entnommen worden sein oder aus Bakterien, Pilzen, mikroskopischen Algen und Protozoen stammen. Das Material kann aus lebenden, fossilen oder mumifizierten Proben, Bodenproben, Kulturen, Konzentraten, Fäkalien, Klärschlamm, Abwässern, forensischen Proben und/oder Lebensmitteln gewonnen worden sein. Es kann sich hierbei um natürlich vorkommendes Zellmaterial ebenso wie um genetisch modifiziertes Zellmaterial handeln.

Als interner Standard wird im Sinne der Erfindung eine Substanz bezeichnet, welche die Erkennung von Schwankungen und Fehlern in der Prozessierung und Analyse der zu untersuchenden Probe ermöglicht. Der interne Standard dient als relative Bezugsgröße, die den Einfluss der Verfahrensparameter auf das Ergebnis eines Verfahrens abbilden und somit eine Validierung der Qualität des Verfahrens erlauben. Im Gegensatz zu einem externen Standard ist ein interner Standard während des gesamten Verfahrens in demselben Gefäß wie die zu untersuchende Probe vorhanden und wird simultan mit dieser prozessiert und analysiert.

Als vollständig in das Reagenz integriert wird der Standard im Sinne der Erfindung bezeichnet, wenn er in einer solchen Art mit dem Reagenz verbunden ist, dass der Benutzer durch alleinige Zugabe des Reagenzes sowohl das Reagenz selbst als auch den Standard zur Probe hinzugibt und kein separater Schritt zur Zugabe des Standards erforderlich ist, wie es bei den aus dem Stand der Technik bekannten Verfahren der Fall ist. Dies wird in der vorliegenden Erfindung dadurch erreicht, dass die Oberfläche der Beads, die zum mechanischen Aufschluss des Probenzellmaterials eingesetzt werden, mit den als Standard verwendeten Referenzzellen wenigstens teilweise beschichtet ist.

Da als Standard ganze Zellen verwendet werden, ermöglicht das erfindungsgemäße Reagenz nicht nur die Kontrolle und Beurteilung des eigentlichen Zellaufschlusses, sondern auch die Kontrolle und Beurteilung nachfolgender Schritte wie der Probenaufbereitung, Extraktion, Anreicherung, Isolierung, Reinigung, reversen Transkription, Amplifikation und Detektion der aus den aufgeschlossenen Zellen erhaltenen Zellbestandteile. Zellbestandteile im Sinne der Erfindung sind bevorzugt Proteine, Ribonukleinsäuren (RNA) und Desoxyribonukleinsäuren (DNA), besonders bevorzugt DNA.

Als Referenzzellen kann eine Vielzahl von Zellen verwendet werden. Die Zellen können pflanzlichen, tierischen und/oder humanen Ursprungs sein, aus Gewebe, Gewebekulturen oder Zellkulturen entnommen worden sein oder aus Bakterien, Pilzen, mikroskopischen Algen und Protozoen stammen. Es kann sich hierbei um natürlich vorkommendes Zellmaterial ebenso wie um genetisch modifiziertes Zellmaterial handeln. Bevorzugt sind die als Referenz verwendeten Zellen natürlich vorkommende und/oder gentechnisch veränderte Mikroorganismen wie Bakterien, Archaea, Pilze, Sporen, Mikroalgen und Protozoen. Besonders bevorzugt im Sinne der Erfindung ist die Verwendung eines Organismus, der unter normalen Umständen nicht (human-)pathogen ist und deshalb ohne besondere Sicherheitsvorkehrungen verschickt und gehandhabt werden kann (z.B. *E. coli*). Das Standardmaterial wird daneben so ausgewählt, dass es in seinen mechanischen Eigenschaften möglichst ähnlich zum Probenmaterial ist. Welches konkrete Referenzzellmaterial als interner Standard verwendet wird, hängt demnach von der Art des Probenzellmaterials ab. Es können auch mehrere Zelltypen gleichzeitig zur Beschichtung der Beads verwendet werden, etwa um dieselbe Referenzmaterial-Präparation für einen breiten Einsatzzweck zusammen mit mehreren Probenmaterialien verwenden zu können.

Die in dem Reagenz verwendeten Beads bestehen aus Glas, Metall, Keramik oder anderen porösen Materialien, oder Wolframcarbid. In einer bevorzugten Ausführungsform sind die Beads Glaskügelchen.

Die Größe der Beads ist abhängig von der Art und der Menge des zu lysierenden Zellmaterials. So erfordert ein effizienter Aufschluss von tierischem oder pflanzlichem Gewebe pro Zeiteinheit im Allgemeinen größere Beads als der Aufschluss von Bakterien oder Hefezellen. Die Größe der Beads beträgt im Sinne der Erfindung 0,01 bis 2 mm, bevorzugt 0,05 mm bis 2 mm, weiterhin bevorzugt 0,1 bis 0,8 mm und insbesondere 0,2 bis 0,6 mm.

Erfindungsgemäß sind die Beads an ihrer Oberfläche mit eingetrockneten Referenzzellen beschichtet. Bevorzugt sind 10² bis 10⁹ Referenzzellen, besonders bevorzugt 10³ bis 10⁸ Referenzzellen und insbesondere 10⁴ bis 10⁷ Reiferenzzellen, bezogen auf eine Menge von 100 mg Beads, auf die Oberfläche der Beads aufgebracht.

Die mit den Referenzzellen beschichtete Oberfläche der Beads kann weiterhin mit einer zusätzlichen Schutzschicht, bspw. aus selbst-assemblierenden Polymeren, versehen sein, um die Referenzzellen zusätzlich zu stabilisieren.

Zum Aufschluss des Zellmaterials wird zu der Probe bevorzugt eine Menge von 10 bis 1000 mg beschichtete Beads pro ml Probenmaterial, besonders bevorzugt eine Menge von 20 bis 800 mg Beads pro ml Probenmaterial und insbesondere eine Menge von 30 bis 600 mg Beads pro ml Probenmaterial hinzugegeben und einem mechanischen Aufschluss zugeführt. Dabei ist die jeweils verwendete Menge der beschichteten Beads pro ml Probenmaterial neben der Stückzahl der Beads auch von der Dichte des verwendeten Beadmaterials abhängig.

Die Erfindung umfasst ferner die Verwendung des erfindungsgemäßen Reagenzes oder der Beads als internen Standard zur Kontrolle und Beurteilung der Vollständigkeit des Aufschlusses von Zellmaterial und nachfolgender Schritte, umfassend wenigstens einen Schritt ausgewählt aus Probensammlung, Probenaufbereitung, Extraktion, Anreicherung, Isolierung, Reinigung, reverser Transkription, Amplifikation und Detektion der aus den aufgeschlossenen Zellen erhaltenen Zellbestandteile oder eine Kombination mehrerer oder aller dieser Schritte, wobei die Beads zum Aufschluss des Zellmaterials verwendet werden. Insbesondere ist das erfindungsgemäße Reagenz zu einer derartigen Verwendung geeignet, in welcher die aus den aufgeschlossenen Zellen erhaltenen Bestandteile Nukleinsäuren, bevorzugt Desoxyribonukleinsäuren darstellen. Die aus dem Referenzzellmaterfal erhaltenen Nukleinsäuren (Referenznukleinsäuren) können beispielsweise nach dem Zellaufschluss gemeinsam mit den Nukleinsäuren des Probenzellmaterials nach dem Fachmann bekannten Verfahren aus der Probe isoliert und gereinigt werden, bspw. unter Verwendung der QIAamp DNA Kits der Firma QIAGEN (Hilden, Deutschland), und mittels einer quantitativen Real Time PCR (qRT-PCR) quantifiziert werden, wobei eine oder mehrere aus den Referenzzellen isolierten Nukleinsäuren für diese dem Aufschluss nachfolgenden Schritte als interner Standard dienen.

Zur Untersuchung werden hierbei selbstverständlich Referenznukleinsäuren ausgewählt, die von den Nukleinsäuren der Probe bspw. anhand der Sequenz unterscheidbar sind. Das Verfahren ist hierbei nicht auf die Untersuchung einer Referenznukleinsäuresequenz beschränkt. Selbstverständlich können auch zwei oder mehr unterschiedliche Nukleinsäurensequenzen der Referenzzellen als interner Standard verwendet werden.

Bei der erfindungsgemäßen Verwendung kann das Probenzellmaterial pflanzlichen, tierischen und/oder humanen Ursprungs sein, aus Gewebe- oder Zellkulturen entnommen worden sein oder aus Bakterien, Pilzen, mikroskopischen Algen und Protozoen stammen. Das Material kann aus lebenden, fossilen oder mumifizierten Proben, Bodenproben, Konzentraten, Kulturen, Präparationen, Fäkalien, Klärschlamm, Abwässern, forensischen Proben und/oder Lebensmitteln gewonnen worden sein. Es kann sich hierbei um natürlich vorkommendes Zellmaterial ebenso wie um genetisch modifiziertes Zellmaterial handeln. Bevorzugt stellt das Probenzellmaterial natürlich vorkommende oder gentechnisch veränderte Mikroorganismen dar.

Die Erfindung stellt weiterhin ein Verfahren zur Herstellung der Referenz-Beads bereit, welches die folgenden Schritte umfasst:
1. Versetzen der Beads mit einer Suspension enthaltend die Referenzzellen,
2. Eintrocknen der Suspension in Gegenwart der Beads,
wobei sich die Referenzzellen, an die Beads anlagern, diese beschichten oder an diese gebunden werden.

Optional kann als das Verfahren zusätzlich einen dritten Schritt des weiteren Beschichtens der eingetrockneten Zellen mit einer Schutzschicht umfassen, bestehend z.B. aus Polymeren, die geeignet sind, die eingetrockneten Zellen zusätzlich gegen vorzeitigen Abbau durch lange Lagerungen bei Umgebungsbedingungen zu schützen.

Bei der die Referenzzellen, enthaltenden Suspension kann es sich um eine Suspension in Wasser, einem Puffer, einem organischen Lösungsmittel, insbesondere Alkohol, einer Salzlösung, einem Nährmedium o. ä. handeln. Die Konzentration der Referenzzellen in der Suspension ist abhängig von der gewünschten Anzahl der Referenzzellen pro Maßeinheit Beads, deren bevorzugter Bereich bereits oben angegeben wurde, sowie dem Volumen der Suspension, das zur Beschichtung der Beads verwendet wird. Im Hinblick auf ein effizientes Beschichten der Beads sollte die Konzentration möglichst hoch gewählt werden, während eine besonders stringente Kontrolle der nachfolgenden Prozessschritte insbesondere dann erreicht wird, wenn die Konzentration an Referenzzellen möglichst gering, bevorzugt im Bereich knapp über der zu erwartenden Nachweisgrenze des nachzuweisenden Biomoleküls aus der Referenzzelle, gewählt wird. Bevorzugt wird ein Verhältnis der Referenzzellsuspension zu den zu beschichtenden Beads (µl/mg) von 0,1 - 10, besonders bevorzugt von 0,15 - 7,5, weiterhin bevorzugt von 0,2 - 5 und insbesondere von 0,25 - 2,5 verwendet.

Das Eintrocknen der Zellsuspension auf den Beads kann durch einfaches Inkubieren der Suspension bei Raumtemperatur (15-30°C), bevorzugt unter sterilen Bedingungen wie unter einer Sterilbank, durch vorsichtiges Erhitzen der Suspension enthaltend die Zellen und die Beads (bevorzugt 30-50°C), ggf. unter leichtem Vakuum oder in einem Inertgasstrom, oder mit einer Reihe weiterer Methoden, die dem Fachmann bekannt sind, erfolgen,

Die Erfindung stellt weiterhin ein Verfahren zum Aufschluss von Zellmaterial unter Verwendung eines vollintegrierten, internen Standards bereit, welches die folgenden Schritte umfasst:
1. Vermengen des erfindungsgemäßen Reagenzes oder der erfindungsgemäßen Beads mit einer Probe enthaltend das zu lysierende Zellmaterial (Probenzellmaterial),
2. simultaner Aufschluss des Proben- und des Referenzzellmaterials durch mechanische Einwirkung auf die Mischung,
wobei die Beads zum Aufschluss des Zellmaterials verwendet werden.

Hierbei kann das Vermengen des erfindungsgemäßen Reagenzes oder der erfindungsgemäßen Beads mit einer Probe, enthaltend das zu lysierende Zellmaterlal (Probenzellmaterial), sowohl durch Zugabe des Reagenzes / der Beads zu der Probe als auch durch das Sammeln der Probe in ein Gefäß erfolgen, welches das Reagenz / die Beads bereits enthält. Insbesondere wenn es sich bei der Probe um festes Zellmaterial wie Gewebe etc. handelt, ist es zwecksmäßig, die Probenzellen vor der Zugabe des Reagenzes in einem geeigneten Puffer zu suspendieren. Der Puffer kann hierbei eine Reihe weiterer Agenzien wie beispielsweise Substanzen zur Stabilisierung von Nukleinsäuren, zur Deaktivierung von Nukleasen und/oder zur Lyse von Proteinen (Proteinasen) enthalten. Auch eher flüssiges Zellmaterial wie Vollblutproben kann vor dem Vermengen mit dem erfindungsgemäßen Reagenz oder den erfindungsgemäßen Beads durch Zugabe von Wasser oder geeigneten Pufferlösungen verdünnt werden. Insbesondere ist es entscheidend, die Proben-Komplexität bzw. die Viskosität der Mischung, enthaltend das Reagenz und die Probe in ihrer Matrix, so einzustellen, dass der mechanische Aufschluss effizient ist. Die interne Kontrolle kann dabei als Standard zur Beurteilung der Eignung der Aufschluss-Bedingungen dienen. Zur Probe wird bevorzugt eine Menge von 10 bis 1000 mg beschichtete Beads pro ml Probenmaterial, besonders bevorzugt eine Menge von 20 bis 800 mg pro ml Probenmaterial und noch weiter bevorzugt eine Menge von 30 bis 600 mg Beads pro ml Probenmaterial hinzugegeben. Dabei ist die Menge abhängig von Material und Dichte, sowie der Größe der verwendeten Beads.

Dem Fachmann sind diverse Verfahren und Geräte bekannt, um unter Verwendung von Beads durch mechanische Einwirkung auf Zellmaterial einen Aufschluss desselben zu erzielen. Exemplarisch seien hier beispielsweise Kugel- und Schwingmühlen, Planetenmühlen, sowie die Verwendung von Ultraschall genannt. Im Sinne der Erfindung erfolgt der Aufschluss des Zellmaterials bevorzugt unter Vibrationsmischen (sogenanntes Vortexen) oder durch Vertikalmischen (z. B: mit dem QIAGEN Tissuelyser), bevorzugt bei Raumtemperatur oder unter vorsichtigem Erhitzen. Die Dauer sowie die Intensität des mechanischen Aufschluss ist abhängig vom aufzuschließenden Zelltyp und der Probenmatrix, was dem Fachmann bekannt ist. Da das Referenzzellmaterial als interner Standard zur Kontrolle der Vollständigkeit des Aufschlusses dient, ist es zweckmäßig, als Referenzzellmaterial Zellen zu verwenden, die unter den gleichen Bedingungen wie das Probenzellmaterial oder schwerer aufzuschließen sind. Die Dauer des Vortexens beträgt bevorzugt 1 bis 20 min, besonders bevorzugt 3 bis 12 min und insbesondere 5 bis 10 min.

Bevorzugt im Sinne der Erfindung wird die Mischung des Referenz- und des Probenzellmaterials zunächst in einem als Prälyse bezeichneten Verfahrensschritt rein mechanisch aufgeschlossen und nach anschließender Zugabe von Proteinasen wie bspw. Proteinase K und gegebenenfalls weiteren chaotropen und/oder Detergenzien enthaltenden Lysepuffem, wie bspw. den Puffern AL oder QSL der Firma QIAGEN (Hilden, Deutschland), durch die kombinierte Wirkung der mechanischen Einwirkung der Beads und der enzymatischen bzw. der chemischen Wirkung des Lysepuffers unter Inaktivierung bzw. enzymatischem Abbau von Proteinen, insbesondere von Nukleasen, lysiert. Auch die bevorzugte Dauer der Prälyse ist abhängig von der aufzuschließenden Probe. Bevorzugt erfolgt die Prälyse durch Vortexen der Mischung aus Proben- und Referenzzellmaterial für 1 bis 20 min, besonders bevorzugt für 3 bis 12 min und insbesondere für 5 bis 10 min auf höchster Stufe, unter Verwendung von geeigneten Probengefäß-Halterungen.

Der Aufschluss des Zellmaterials kann ferner auch unter Verwendung einer Mischung der erfindungsgemäßen beschichteten Beads mit unbeschichteten Beads des gleichen oder eines anderen Materials erfolgen, wobei die unbeschichteten Beads die gleiche oder eine andere Größe als die beschichteten Beads haben können und auch eine Mischung unbeschichteter Beads unterschiedlicher Größe umfassen können.

Die Erfindung umfasst auch einen Kit zum Nachweis von Nukleinsäuren in Zellmaterial, enthaltend das erfindungsgemäße Reagenz oder die erfindungsgemäßen Beads sowie optional geeignete Primer zur Amplifikation der Proben- und/oder Referenznukleinsäuren in nachfolgenden PCR-Verfahren oder anderen Nukleinsäure-Amplifikations-Technologien oder Nukleinsäure-Detektions-Technologien. Besonders geeignet für den Ansatz der Erfindung sind hierbei sogenannte quantitative Echtzeit-PCR-Verfahren (qRT-PCR) oder qualitative bzw. quantitative Isothermale Amplifikationstechnologien.

Alternativ kann das Referenzreagenz auch in einer ausreichend hohen Konzentration eingesetzt werden, so dass es in einer nachfolgenden Nachweisreaktion ohne Amplifikation detektiert werden kann. Bei einer solchen Verwendung des Referenzreagenzes wird spezifisch der Prozess des Probenaufschlusses und der Proben-Vorbereitung sowie der Isolation des nachzuweisenden Zielmoleküls kontrolliert, während die Amplifikationsreaktion nicht kontrolliert wird. Dies kann z.B. von Vorteil sein, wenn gezielt einzelne Prozess-Schritte kontrolliert werden sollen.

Als Primer wird Im Sinne der Erfindung ein Oligonukleotid verstanden, welches mit einer Nukleinsäuresequenz (aus dem Referenz- oder dem Probenzellmaterial) unter Ausbildung einer doppelsträngigen Region hybridisieren kann. Diese doppelsträngige Region kann als Ausgangspunkt der Amplizifierung beispielsweise per qRT-PCR nach Verfahren, die dem Fachmann bekannt sind, genutzt werden.

Durch die in den nachfolgenden Beispielen näher erläuterten Versuche konnte gezeigt werden, dass sich die erfindungsgemäßen Beads sowohl als Kontrolle zur Vollständigkeit des Aufschlusses des Zellmaterials als auch als interner Standard für nachfolgende Untersuchungen einzelner Zeilbestandtelle wie beispielsweise mit Hilfe von DNA der qRT-PCR eignen. Ferner konnte gezeigt werden, dass es möglich ist, mit Referenzzellen beschichtete Beads herzustellen, deren Zellen selbst nach einer Lagerung von mehr als einem Jahr bei Raumtemperatur größtenteils noch intakt vorlagen und somit als interner Standard zur Kontrolle eines Zellaufschlusses und nachfolgender Schritte dienen können.

### Beschreibung der Abbildungen

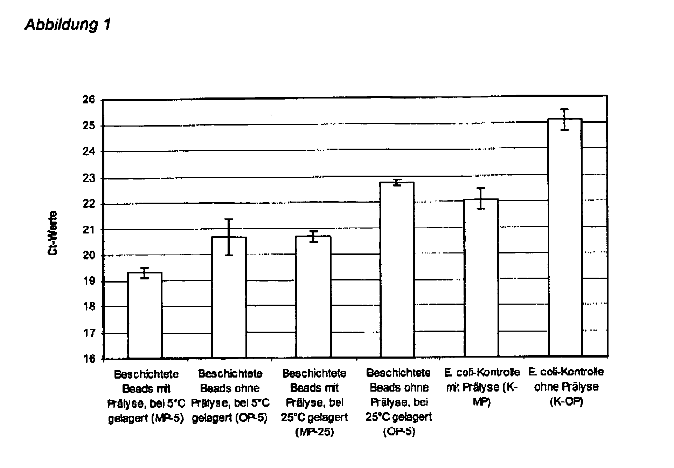

Abbildung 1 zeigt die mittels qRT-PCR bestimmten C_{T}-Werte der Amplifikation von *E*. *coli* DNA, welche durch mechanische Einwirkung unter Verwendung von Glas-Beads aufgeschlossen wurden.

### Beispiele

### Beispiel 1: Herstellung der mit Referenzzellen beschichteten Beads

Eine *E. coli-*Kultur wurde in einem LB-Nährmedium (Lysogeny Broth-Medium, G. Bertani J. Bacteriol., 1951, 62(3), 293 - 300) angezogen. Mithilfe einer Zählkammer wurde unter dem Mikroskop die Zellenanzahl in diesem Medium zu 3.03 x 10⁹ pro ml bestimmt. Die Kultur wurde mit weiterem LB-Medium auf eine Konzentration von 2 x 10⁷ Zellen pro ml verdünnt. 1 ml dieser Zellsuspension wurde abgenommen, und die Zellen wurden durch Zentrifugation pelletiert. Das erhaltene Pellet wurde in 1 ml einer 0.9 % NaCl-Lösung (aq) suspendiert. Jeweils 50 µl dieser Suspension wurden auf 100 mg Glas-Beads (600 µm, Sigma G8772, Lot-Nr. 100 K5339) gegeben, und die Lösung wurde über Nacht unter einer Sterilbank eintrocknen gelassen. Die Anzahl der Zellen betrug somit 1 x 10⁶ Zellen pro 100 mg Glas-Beads.

### Beispiel 2: Lyse von Vollblut

Jeweils fünf Chargen (je 100 mg) der gemäß Beispiel 1 erhaltenen beschichteten Beads wurden für 1 Jahr bei 2 - 8 °C und 25 ± 3 °C gelagert. Anschließend wurden die gelagerten Beads in humanes Vollblut eingebracht, und die beschichteten Bakterienzellen wurden in dieser biologischen Matrix durch mechanische Lyse aufgeschlossen. Nach dieser Prälyse wurde eine QIAGEN Sample Preparation unter Verwendung des unten beschriebenen QIAamp DNA Blood Mini Kits (QIAGEN, Hilden, Deutschland) gemäß des "QIAamp DNA Mini Kit and QIAamp DNA Blood Mini Kit" Handbuches 11/2007 angeschlossen, um die DNA der aufgeschlossenen Bakterienzellen und die aus den Leukozyten im Blut stammende humane DNA zu isolieren und aufzurelnigen.

Die als Prälyse bezeichnete mechanische Aufschluss-Prozedur wurde in vorangegangenen Versuchen dahingehend optimiert, dass im Probenmaterial Blut ein optimaler Aufschluss von Bakterien und Pilz-Zellen erreicht wurde. In Blut wurde ein optimaler Aufschluss durch fünfminütiges Vortexen der Probe mit den Glass Beads in einer 2:1 Mischung aus Blut und Prälysepuffer (enthaltend Tris, EDTA und Triton X-100) erreicht. Die nachfolgenden Versuche zur Untersuchung der Referenz-Beads wurden daher mit dem gleichen Prälyse-Protokoll durchgeführt, um die Aufschluss-Effizienz der auf den Beads eingetrockneten Bakterienzellen zu ermitteln. Zu Vergleichszwecken wurden die Analysen mit und ohne Prälyse durchgeführt. Dieser Vergleich sollte insbesondere zeigen, ob die Prälyse bei Verwendung der beschichteten Beads, welche bereits über ein Jahr gelagert wurden, noch erforderlich ist, oder ob ggf. nach einem Jahr Lagerung die auf den Beads aufgetrockneten Zellen nicht mehr intakt sind und die DNA demzufolge bereits frei vorliegen würde, was eine Prälyse überflüssig machen würde, und damit die Kontrollfunktion der Referenz-Beads eliminieren würde. Des Weiteren wurden Kontrollen mitgeführt, die die gleiche Menge Bakterien, die auf den beschichteten Beads aufgetrocknet wurden, frisch eingespickt in Blut enthielten.

Der Zellaufschluss wurde mit folgenden Proben durchgeführt:
1. Drei Chargen der bei 5°C gelagerten Beads (jeweils 100 mg) und jeweils 600 µl frisch aufgetautes Blut mit Prälyse (im Folgenden als MP-5 bezeichnet);
2. drei Chargen der bei 25°C gelagerten Beads (jeweils 100 mg) und jeweils 600 µl frisch aufgetautes Blut mit Prälyse (MP-25);
3. zwei Chargen der bei 5°C gelagerten Beads (jeweils 100 mg) und jeweils 600 µl frisch aufgetautes Blut ohne Prälyse (OP-5);
4. zwei Chargen der bei 25°C gelagerten Beads (jeweils 100 mg) und jeweils 600 µl frisch aufgetautes Blut ohne Prälyse (OP-25).
5. drei Chargen einer Kontrollprobe aus 200 mg unbeschichteten Beads und jeweils 600 µl Blut, das mit 1,07 x 10⁶ Zellen *E*. *coli* versetzt wurde mit Prälyse (K-MP);
6. drei Chargen einer Kontrollprobe aus 200 mg unbeschichteten Beads und jeweils 600 µl des unter 5. beschriebenen *E*. *coli-*gespickten Blutes ohne Prälyse (K-OP);
7. jeweils drei Kontrollproben aus 600 µl ungespicktem Blut (K-B).

Zur Herstellung der *E. coli*-gespickten Blutproben wurden 10 ml Blut mit 143 µl einer *E. coli*-Glycerin-Stockkultur, die 1,25 x 10⁸ Zellen pro ml enthielt, versetzt.

Zum Aufschluss der Zellen wurden 600 µl frisch aufgetautes Blut (für die Proben MP-5, OP-5, MP-25, OP-25, K-B) bzw. 600 µl *E. coli*-gespiktes Blut (für die Proben K-OP, K-MP) In 300 µl SPL1-Puffer zu 200 mg der unbehandelten Beads (K-OP, K-MP, K-B) bzw. 100 mg der beschichteten Beads (MP-5, OP-5, MP-25, OP-25) in einem 1,5 ml-Probengefäß der Firma Eppendorf gegeben. Nachfolgend wurden die Proben mit Prälyse (MP-5, MP-25, K-MP) fünf Minuten auf höchster Stufe gevortext. bzw. fünf Minuten bei Raumtemperatur inkubiert (Proben ohne Prälyse). 600 µl des Überstandes wurden nach Absinken der Glassbeads in ein neues Probengefäß überführt und nach Zugabe von 60 µl Proteinase K kurz durch Vortexen gemischt. Nach Zugabe von 450 µl AL-Puffer der Firma QIAGEN wurde die Probe weitere 15 Sekunden gevortext. Die oben beschriebenen Schritte wurden unter einer Sterilbank durchgeführt. Die Proben wurden dann jeweils zehn Minuten bei 56°C inkubiert und danach kurz zentrifugiert, mit 750 µl einer Mischung von 80% Ethanol und 20% AL-Puffer der Firma QIAGEN (Hilden, Deutschland) versetzt und 15 Sekunden gevortext. Die gesamten Ansätze wurden jeweils in Extender-Tubes der Firma QIAGEN (Hilden, Deutschland) überführt, die auf QIAamp Minisäulen der Firma QIAGEN (Hilden, Deutschland) aufgesteckt wurden. Das Lysat wurde dann unter Vakuum durch die QIAamp-Minisäulen gezogen, wobei die freigesetzte Nukleinsäure an die Silika-Matrix der Säule bindet. Anschließend wurde die Säule zweimal mit je 700 µl AW1-Puffer der Firma QIAGEN (Hilden, Deutschland) gewaschen. Die Entfernung der Waschlösung erfolgte unter reduziertem Druck. Danach wurde die Säule zweimal mit je 750 µl des AW2-Puffers der Firma QIAGEN (Hilden, Deutschland) gewaschen, und die Waschlösung wurde unter reduziertem Druck entfern. Anschließend wurde die QIAamp-Minisäule von der Vakuumvorrichtung abgenommen, in ein neues Probengefäß überführt und durch dreiminütiges Zentrifugieren bei 15.000 G getrocknet. Die Säule wurde in ein neues Gefäß überführt und zusätzlich fünf Minuten bei 56°C im Heizblock getrocknet. Die DNA wurde anschließend mit 100 µl Reinstwasser eluiert, wobei eine Minute auf der Membran der Spin Columns bei Raumtemperatur inkubiert wurde, bevor das Eluat durch Zentrifugation bei 15.000 G für 1 Minute gewonnen wurde. Die erhaltenen Eluate wurden photometrisch untersucht, um die Gesamt-DNA-Ausbeute, also die aus dem Blut erhaltene humane DNA inklusive der *E*. *coli*-DNA, zu bestimmen, wobei der Anteil der aus dem Referenzzellmaterial erhaltenen DNA gegenüber der aus der Probe erhaltenen DNA gering ist. Die Ergebnisse sind in Tabelle 1 dargestellt. Des Weiteren wurde eine qRT-PCR-Analyse zum quantitativen spezifischen Nachweis der E. coli-DNA durchgeführt.

**Tabelle 1:**

| Probe | Charge | A260 | A260/ A280 | Ausbeute µg/Elution | | | |
|---|---|---|---|---|---|---|---|
| | | | | [µg] | Ø | SD¹ | CV² % |
| beschichtete Beads mit Prälyse, bei 5°C gelagert (MP-5) | 1 | 0,30 | 1,95 | 8,49 | 7,7 | 0,8 | 9,9 |
| | 2 | 0,27 | 1,93 | 7,80 | | | |
| | 3 | 0,24 | 1.93 | 6,66 | | | |
| beschichtete Beads mit Prälyse, bei 25°C gelagert (MP-25) | 1 | 0,26 | 1,92 | 7,26 | 7,5 | 1,2 | 15,9 |
| | 2 | 0,33 | 1.94 | 9,03 | | | |
| | 3 | 0,23 | 1.90 | 6,15 | | | |
| beschichtete Beads ohne Prälyse, bei 5°C gelagert (OP-5) | 1 | 0,23 | 1,95 | 6,78 | 5,9 | 0,9 | 15,0 |
| | 2 | 0,17 | 1,88 | 5,01 | | | |
| beschichtete Beads ohne Prälyse, bei 25°C gelagert (OP-25) | 1 | 0,19 | 1,91 | 5,40 | 5,6 | 0,2 | 4,3 |
| | 2 | 0,21 | 1,88 | 5.88 | | | |
| Kontrolle E. coli mit Prälyse (K-MP) | 1 | 0,18 | 2,68 | 3,76 | 4,3 | 0,5 | 11,8 |
| | 2 | 0,17 | 1,93 | 4,98 | | | |
| | 3 | 0,14 | 1,93 | 4,11 | | | |
| Kontrolle E. coli ohne Prälyse (K-OP) | 1 | 0,20 | 1,91 | 5,85 | 5,6 | 0,2 | 4,1 |
| | 2 | 0,20 | 1,92 | 5,70 | | | |
| | 3 | 0,18 | 1,82 | 5,31 | | | |
| Kontrolle Blut (K-B) | 1 | 0,20 | 1,90 | 5,58 | 4,9 | 0.7 | 14,5 |
| | 2 | 0,17 | 1,88 | 5,07 | | | |
| | 3 | 0,13 | 1,86 | 3,90 | | | |

| | | | | | | | |
|---|---|---|---|---|---|---|---|
| ¹ SD: Standardabweichung: ²CV: Variationskoeffizient | | | | | | | |

Die Menge der Gesamt-DNA wurde durch photometrische Bestimmungen der Lichtabsorption bei einer Wellenlänge von 260 nm ermittelt. Es ist deutlich zu erkennen, dass die Verwendung der beschichteten Beads und auch die Prälyse-Prozedur, also das Vortexen der mit den Beads vermengten Probe, keinen negativen Einfluss auf die Gesamtausbeute an DNA haben.

Die Reinheit der erhaltenen DNA wurde über die photometrische Bestimmung des Verhältnisses der Lichtabsorption bei 260 nm zu derjenigen bei 280 nm (A260/A280) bestimmt. Das Verhältnis A260/A280 lag für alle Proben, die unter Verwendung der beschichteten Beads aufgeschlossen wurden im Bereich zwischen 1.8 und 2.0. Folglich hat die mehr als einjährige Lagerung der beschichteten Proben keinen Einfluss auf die Reinheit der erhaltenen DNA.

### Beispiel 3: qRT-PCR-Analyse zum spezifischen Nachweis der E. coli-DNA

Um nachzuweisen, dass auch nach einer einjährigen Lagerung die *E. coli*-DNA aus den beschichteten Beads noch intakt und nachweisbar ist, wurde eine Echtzeit-Polymerasekettenreaktion (qRT-PCR) zum Nachweis der *E. coli-*DNA in den in Beispiel 2 beschriebenen Proben MP-5, OP-5, MP-25, OP-25, K-MP und K-OP durchgeführt. Die Reaktionen wurden unter Verwendung der FRET-Sonde 5' [6∼FAM]CACTACGGTGCTGAAGCGACAAA[BHQ1a∼BFAM] und der Primer 5'CCAGGCAAATCCGGAAAAC3' und 5'GTACGATTTGATGTTACCTGAT3' an einem ABI Taqman 7900 (Applied Biosystems Inc., Foster City, Kalifornien, USA) in Ansätzen zu jeweils 25 µl (10 µl Templat, 0.6 µl einer 10 µM Lösung der FAM-BHQ-funktionalisierten FRET-Sonde, 12.5 µl QIAGEN 2x QuantiTect Multiplex PCR, 0,6 µl eines Gemisches von jeweils 10 µM Lösungen der beiden Primer und 1,3 µl Reinstwasser). Insgesamt wurden 40 Zyklen durchgeführt, wobei ein Zyklus jeweils die folgenden Schritte umfasste: Denaturierung (15 min bei 95°C), Annealing (45 s bei 94°C) und Elongation (75 s bei 60°C).

Die so ermittelten C_{T}-Werte (Threshold Cycle, Schwellenwertzyklus) sind in Tabelle 2 dargestellt.

**Tabelle 2:**

| Probe | C_{T} Werte | | | |
|---|---|---|---|---|
| | C_{T} | Ø | SD¹ | CV² [%] |
| beschichtete Beads mit Prälyse, bei 5°C gelagert (MP-5) | 19,49 | 19,31 | 0,20 | 1,04 |
| | 19,35 | | | |
| | 19,09 | | | |
| geschichtete Beads mit Prälyse, bei 25°C gelagert (MP-25) | 20,85 | 20,67 | 0,20 | 0,95 |
| | 20,46 | | | |
| | 20,70 | | | |
| beschichtete Beads ohne Prälyse, bei 5°C gelagert (OP-5) | 20,87 | 20,36 | 0,72 | 3,54 |
| | 19,85 | | | |
| beschichtete Beads ohne Prälyse, bei 25°C gelagert (OP-25) | 22,85 | 22,75 | 0,14 | 0,60 |
| | 22,65 | | | |
| Kontrolle E. coli mit Prälyse (K-MP) | 21,65 | 22,08 | 0,37 | 1,67 |
| | 22,29 | | | |
| | 22,29 | | | |
| Kontrolle E. coli ohne Prälyse (K-OP) | 24,99 | 25,02 | 0,40 | 1,58 |
| | 24,64 | | | |
| | 25,43 | | | |

| | | | | |
|---|---|---|---|---|
| ¹ SD: Standardabweichung; ² CV: Variationskoeffizient | | | | |

Auch wenn ein direkter Vergleich zwischen den Proben, die mit den beschichteten Beads behandelt wurden, und denen, die mit *E. coli-*Zellen gespickt wurden, aufgrund der unterschiedlichen Ausgangskulturen von *E. coli*-Bakterien schwierig ist, ist dennoch deutlich zu erkennen, dass selbst nach einjähriger Lagerung die *E. coli*-DNA auf den beschichteten Beads mittels qRT-PCR noch sehr gut amplifizierbar und nachweisbar war. Des Weiteren ist zu erkennen, dass die Prälyse einen signifikant positiven Einfluss auf den in einer nachfolgenden Amplifikation ermittelten C_{T}-Wert hat, was darauf hindeutet, dass ein Großteil der Zellen noch intakt vorlag und eines Aufschlusses bedurfte. Dies ist auch in Abbildung 1 verdeutlicht, die die mittels qRT-PCR ermittelten C_{T}-Werte in Abhängigkeit vom Aufschlussverfahren zeigt. Die C_{T}-Werte derjenigen Blutproben, welche unter Verwendung der beschichteten Beads aufgeschlossen wurden (MP-5, MP-25 bzw. OP-5, OP-25), liegen unter dem C_{T}-Wert, welcher für die aus der reinen Blutprobe (K-MP bzw. K-OP) erhaltene DNA ermittelt wurde, und zwar sowohl für die bei 5°C gelagerten Beads als auch für die bei 25°C gelagerten Beads. Untereinander zu vergleichen sind hierbei jeweils die nach dem gleichen Verfahren behandelten Proben, also diejenigen mit Prälyse (MP) bzw. ohne Prälyse (OP). Ein Vergleich der Proben, die einer Prälyse unterworfen wurden, mit denjenigen, die ohne Prälyse aufgeschlossen wurden, zeigt ferner, dass die Verwendung des Prälyse-Schrittes einen signifikant positiven Einfluss auf den erhaltenen C_{T}-Wert hat, wenn zum Zellaufschluss die beschichteten Beads eingesetzt werden, was darauf hindeutet, dass ein Großteil der *E. coli*-Zellen in der Beschichtung selbst nach einer Lagerung von mehr als einem Jahr noch intakt vorlag und einen Aufschluss zuließ.

Dies traf sowohl auf die bei 25°C gelagerten wie auch auf die bei 5°C gelagerten beschichteten Beads zu. Zusammenfassend zeigen die Beispiele 2 und 3, dass die auf Glas-Beads aufgebrachten Zellen über einen langen Zeitraum (t > 1 Jahr) stabil sind und auch noch nach einjähriger Lagerung als Kontrolle für die Vollständigkeit des Aufschlusses sowie zur Beurteilung nachfolgender Untersuchungen wie beispielsweise einer qRT-PCR dienen können.

## Patentansprüche

1. Reagenz zum Aufschluss von Zellmaterial (Probenzellmaterial), enthaltend einen vollständig in das Reagenz integrierten internen Standard zur Kontrolle und Beurteilung der Vollständigkeit des Aufschlusses des Zellmaterials und nachfolgender Schritte, umfassend einen Schritt ausgewählt aus Probenaufbereitung, Extraktion, Anreicherung, Isolierung, Reinigung, reverse Transkription, Amplifikation und Detektion der aus den aufgeschlossenen Zellen erhaltenen Zellbestandteile oder einer Kombination mehrerer oder aller dieser Schritte, umfassend Beads, deren Oberfläche mit eingetrockneten Referenzzellen (Referenzzellmaterial) als Standard wenigstens teilweise beschichtet ist, wobei die Beads aus Glas, Metall, Keramik oder anderen porösen Materialien oder Wolf ramcarbid bestehen und die Größe der Beads 0,01 mm bis 2 mm beträgt und wobei die Beads durch Eintrocknen einer Suspension, die diese Referenzzellen enthält, in Gegenwart der Beads hergestellt wurden, wodurch sich die Zeilen an die Beads anlagern, diese beschichten oder an diese gebunden werden.

2. Reagenz gemäß Anspruch 1, wobei die als Referenz verwendeten Zellen natürlich vorkommende und/oder gentechnisch veränderte Mikroorganismen sind.

3. Reagenz gemäß Anspruch 1 oder 2, wobei die Beads Glaskügelchen sind.

4. Beads, deren Oberfläche mit eingetrockneten Referenzzellen wenigstens teilweise beschichtet ist, wobei die Beads aus Glas, Metall, Keramik oder anderen porösen Materialien oder Wolframcarbid bestehen und die Größe der Beads 0,01 mm bis 2 mm beträgt, wobei die Beads durch Eintrocknen einer Suspension, die diese Referenzzellen enthält, in Gegenwart der Beads hergestellt wurden, wodurch sich die Zellen an die Beads anlagern, diese beschichten oder an diese gebunden werden.

5. Beads gemäß Anspruch 4, wobei die Größe der Beads 0,05 mm bis 2 mm, bevorzugt 0,1 bis 0,8 mm und besonders bevorzugt 0,2 bis 0,6 mm beträgt.

6. Beads gemäß Anspruch 4 oder 5, wobei 10² bis 10⁹ Referenzzellen, bevorzugt 10³ bis 10⁸ Referenzzellen und besonders bevorzugt 10⁴ bis 10⁷ Referenzzellen auf die Oberfläche von 100 mg Beads aufgebracht sind.

7. Beads gemäß einem der Ansprüche 4 bis 6, wobei die mit den Referenzzellen wenigstens teilweise beschichtete Oberfläche der Beads mit einer zusätzlichen Schutzschicht versehen ist.

8. Verwendung des Reagenzes gemäß irgendeinem der Ansprüche 1 bis 3 oder der Beads gemäß irgendeinem der Ansprüche 4 oder 7 als internen Standard zur Kontrolle und Beurteilung der Vollständigkeit des Aufschlusses des Zellmaterials und nachfolgender Schritte, umfassend einen Schritt ausgewählt aus Probenaufbereitung, Extraktion, Anreicherung, Isolierung, Reinigung, reverse Transkription, Amplifikation und Detektion der aus den aufgeschlossenen Zellen erhaltenen Zellbestandteile oder eine Kombination mehrerer oder aller dieser Schritte, wobei die Beads zum Aufschluss des Zellmaterials verwendet werden.

9. Verwendung gemäß Anspruch 8, wobei die aus den aufgeschlossenen Zellen erhaltenen Bestandteile Nukleinsäuren, bevorzugt Desoxyribonukleinsäuren darstellen.

10. Verwendung gemäß Anspruch 8 oder 9, wobei das Probenzellmaterial natürlich vorkommende oder gentechnisch veränderte Mikroorganismen darstellt.

11. Verfahren zur Herstellung der Beads gemäß irgendeinem der Ansprüche 4 bis 7, umfassend wenigstens die folgenden Schritte:
(1) Versetzen der Beads mit Suspension enthaltend die Referenzzellen,
(2) Eintrocknen der wässrigen Lösung auf den Beads und
(3) optional Beschichten der Beads, welche auf ihrer Oberfläche die eingetrockneten Referenzzellen tragen, mit einer Schutzschicht.

12. Verfahren zum Aufschluss von Zellmaterial unter Verwendung eines vollintegrierten, internen Standards, umfassend die folgenden Schritte:
(1) Vermengen des Reagenzes gemäß irgendeinem der Ansprüche 1 bis 3 oder der Beads gemäß irgendeinem der Ansprüche 4 bis 7 mit einer Probe, enthaltend das aufzuschließende Zellmaterial,
(2) Simultaner Aufschluss des Proben- und des Referenzzellmaterials durch mechanische Einwirkung auf die Mischung,
wobei die Beads zum Aufschluss des Zellmaterials verwendet werden.

13. Verfahren gemäß Anspruch 12, wobei der Aufschluss des Zellmaterials unter Vibrationsmischen (Vortexen) vorgenommen wird.

14. Verfahren gemäß Anspruch 12 oder 13, wobei der Aufschluss des Zeilmaterials unter Verwendung einer Mischung der beschichteten Beads gemäß irgendeinem der Ansprüche 4 bis 6 mit unbeschichteten Beads erfolgt.

15. Kit zum Nachweis von Nukleinsäuren in Zellmaterial enthaltend:
(1) Reagenz gemäß irgendeinem der Ansprüche 1 bis 3 oder Beads gemäß irgendeinem der Ansprüche 4 oder 7,
(2) Primer zur Amplfikation der Proben- oder der Referenznukleinsäuren.

## Claims

1. A reagent for digesting cell material (sample cell material), containing an internal standard for monitoring and assessing the digestion of the cell material and subsequent steps, completely integrated into the reagent, comprising a step selected from sample preparation, extraction, enrichment, isolation, cleaning, reverse transcription, amplification and detection of the cell components obtained from the open cells or a combination of several or all of these steps, comprising beads which, as a standard, have the surface at least partially coated with dried up reference cells (reference cell material), **characterised in that** the beads consist of glass, metal, porcelain or other porous materials or tungsten carbide and the size of the beads amounts to 0.01 mm to 2 mm, and **characterised in that**, the beads have been created by drying out a suspension containing such reference cells in the presence of the beads, as a result of which the cells attach to the beads, coat them or are bonded to them.

2. The reagent in accordance with Claim 1, **characterised in that** the cells used as a reference are naturally occurring and/or genetically modified micro-organisms.

3. The reagent in accordance with Claim 1 or 2, **characterised in that** the beads are glass pellets.

4. Beads, the surface of which is at least partially coated with dried up reference cells, **characterised in that** the beads consist of glass, metal, porcelain or other porous materials or tungsten carbide and the size of the beads amounts to 0.01 mm to 2 mm, wherein the beads have been created by drying out a suspension containing these reference cells in the presence of the beads, as a result of which the cells attach to the beads, coat them or are bonded to them.

5. Beads in accordance with Claim 4, **characterised in that** the size of the beads amounts to 0.05 mm to 2 mm, preferably 0.1 to 0.8 mm and particularly preferably 0.2 to 0.6 mm.

6. Beads in accordance with Claim 4 or 5, **characterised in that** 10² to 10⁹ reference cells, preferably 10³ to 10⁸ reference cells and particularly preferably 10⁴ to 10⁷ reference cells are applied to the surface of 100 mg beads.

7. Beads in accordance with one of Claims 4 to 6, **characterised in that** the surface of the beads that is at least partially coated with the reference cells is provided with an additional protective layer.

8. Use of the reagent in accordance with any of Claims 1 to 3 or the beads in accordance with either of Claims 4 or 7 as an internal standard for monitoring and assessing the completeness of the openness of the cell material and subsequent steps, comprising a step selected from sample preparation, extraction, enrichment, isolation, cleaning, reverse transcription, amplification and detection of the cell components obtained from the open cells or a combination of several or all of these steps, wherein the beads are used to digest the cell material.

9. Use in accordance with Claim 8, **characterised in that** the components obtained from the open cells consist of nucleic acids, preferably deoxyribonucleic acids.

10. Use in accordance with Claim 8 or 9, **characterised in that** the sample cell material consists of naturally occurring or genetically modified micro-organisms.

11. A method of creating the beads in accordance with any one of Claims 4 to 7, comprising at least the following steps:
(1) Adding suspension containing the reference cells to the beads;
(2) Drying out the aqueous solution on the beads; and
(3) Optionally coating the beads which bear the dried out reference cells on their surface with a protective layer.

12. A method of digesting cell material using a fully-integrated internal standard, comprising the following steps:
(1) Admixing the reagent in accordance with one of Claims 1 to 3 or the beads in accordance with any of Claims 4 to 7 with a sample, containing the cell material to be digested;
(2) Simultaneous digestion of the sample and reference cell material through mechanical impact on the formulation,
**characterised in that** the beads are used to digest the cell material.

13. A method in accordance with Claim 12, **characterised in that** the digestion of the cell material is performed using vibration mixing (vortexing).

14. A method in accordance with Claim 12 or 13, **characterised in that** the cell material is digested using a mixture of the coated beads in accordance with any of Claims 4 to 6 with uncoated beads.

15. A kit for evidencing nucleic acids in cell material containing:
(1) The reagent in accordance with one of Claims 1 to 3 or beads in accordance with either of Claims 4 or 7,
(2) A primer for amplification of the sample or reference nucleic acids.

## Revendications

1. Réactif de dissociation de matériel cellulaire (matériel cellulaire d'échantillon), contenant un étalon interne entièrement intégré dans le réactif pour le contrôle et l'évaluation du degré d'achèvement de la dissociation du matériel cellulaire et des étapes suivantes, comprenant une étape choisie entre la préparation d'un échantillon, l'extraction, l'enrichissement, l'isolation, la purification, la transcription inverse, l'amplification et la détection des constituants cellulaires provenant des cellules dissociées ou une combinaison de plusieurs ou de l'ensemble de ces étapes, comportant des billes, dont la surface est au moins partiellement revêtue de cellules de référence déshydratées (matériel cellulaire de référence) en tant qu'étalon, les billes étant en verre, métal, céramique ou en autre matériau poreux ou en carbure de tungstène, la grandeur des billes étant comprise entre 0,01 mm et 2 mm, et les billes ayant été produites par déshydratation d'une suspension contenant lesdites cellules de référence en présence des billes, les cellules s'agglomérant ainsi contre les billes, revêtant celles-ci ou leur étant liées.

2. Réactif selon la revendication 1, où les cellules servant de référence sont des microorganismes rencontrés naturellement et/ou génétiquement modifiés.

3. Réactif selon la revendication 1 ou la revendication 2, où les billes sont des perles de verre.

4. Billes, dont la surface est au moins partiellement revêtue de cellules de référence déshydratées, les billes étant en verre, métal, céramique ou en autre matériau poreux ou en carbure de tungstène, et la grandeur des billes étant comprise entre 0,01 mm et 2 mm, les billes ayant été étant produites par déshydratation d'une suspension contenant lesdites cellules de référence en présence des billes, les cellules s'agglomérant ainsi contre les billes, revêtant celles-ci ou leur étant liées.

5. Billes selon la revendication 4, où la grosseur des billes est comprise entre 0,05 mm et 2 mm, préférentiellement entre 0,1 et 0,8 mm et tout particulièrement entre 0,2 et 0,6 mm.

6. Billes selon la revendication 4 ou 5, où 10² à 10⁹ cellules de référence, préférentiellement 10³ à 10⁸ cellules de référence et tout particulièrement 10⁴ à 10⁷ cellules de référence sont appliquées à la surface de 100 mg de billes.

7. Billes selon l'une des revendications 4 à 6, où la surface des billes au moins partiellement revêtue des cellules de référence est pourvue d'une couche protectrice supplémentaire.

8. Utilisation du réactif selon l'une quelconque des revendications 1 à 3 ou des billes selon l'une quelconque des revendications 4 ou 7 en tant qu'étalon interne pour le contrôle et l'évaluation du degré d'achèvement de la dissociation du matériel cellulaire et des étapes suivantes, comprenant une étape choisie entre la préparation d'un échantillon, l'extraction, l'enrichissement, l'isolation, la purification, la transcription inverse, l'amplification et la détection des constituants cellulaires provenant des cellules dissociées ou une combinaison de plusieurs ou de l'ensemble de ces étapes, où les billes sont utilisées pour la dissociation du matériel cellulaire.

9. Utilisation selon la revendication 8, où les constituants cellulaires provenant des cellules dissociées constituent des acides nucléiques, préférentiellement des acides désoxyribonucléiques.

10. Utilisation selon la revendication 8 ou 9, où le matériel cellulaire d'échantillon constitue des microorganismes rencontrés naturellement ou génétiquement modifiés.

11. Procédé de fabrication de billes selon l'une quelconque des revendications 4 à 7, comprenant au moins les étapes suivantes :
(1) mélange des billes avec la suspension contenant les cellules de référence,
(2) déshydratation de la solution aqueuse sur les billes et
(3) revêtement facultatif d'une couche protectrice sur les billes portant les cellules de référence déshydratées à leur surface.

12. Procédé de dissociation de matériel cellulaire par utilisation d'un étalon interne entièrement intégré, comprenant les étapes suivantes :
(1) mélange du réactif selon l'une quelconque des revendications 1 à 3 ou des billes selon l'une quelconque des revendications 4 à 7 avec un échantillon contenant le matériel cellulaire à dissocier,
(2) dissociation simultanée du matériel cellulaire d'échantillon et du matériel cellulaire de référence par action mécanique sur le mélange,
les billes étant utilisées pour la dissociation du matériel cellulaire.

13. Procédé selon la revendication 12, où la dissociation du matériel cellulaire est effectuée par mélange par vibrations (par Vortex).

14. Procédé selon la revendication 12 ou 13, où la dissociation du matériel cellulaire est effectuée en recourant à un mélange des billes revêtues selon l'une quelconque des revendications 4 à 6 avec des billes non revêtues.

15. Kit pour la d'acides nucléiques dans du matériel cellulaire, contenant :
(1) un réactif selon l'une quelconque des revendications 1 à 3 ou des billes selon l'une quelconque des revendications 4 ou 7,
(2) une amorce pour l'amplification des acides nucléiques d'échantillon ou de référence.
